# EUROPÄISCHE PATENTANMELDUNG

(11) **EP 4 740 913 A2**
(43) Veröffentlichungstag der Anmeldung: **13.05.2026**
(21) Anmeldenummer: 26158467.6
(22) Anmeldetag: 17.11.2022
(51) Int. Cl.: A61F 2/68

(54) **PROTHESENHANDGELENK**

(30) Priorität: 23.11.2021 DE 102021130659
(62) Teilanmeldung aus: 22818668.0
(71) Anmelder: Otto Bock Healthcare Products GmbH, 1110 Wien (AT)
(72) Erfinder: HASLINGER, Martin, 1110 Wien (AT); MITTELBERGER, Felix, 1110 Wien (AT); SAGMEISTER, Luis, 1110 Wien (AT)
(74) Vertreter: Gramm, Lins & Partner Patent- und Rechtsanwälte PartGmbB

(57) **Zusammenfassung**

Die Erfindung betrifft ein Prothesenhandgelenk mit einer Basis (10) zur Befestigung an einer proximalen Prothesenkomponente und einem Schwenkteil (20), das um eine Schwenkachse (30) verschwenkbar an der Basis (10) gelagert ist,
- mit zumindest einer Befestigungseinrichtung (21) zur Festlegung einer distalen Prothesenkomponente oder Prothesenhand an dem Schwenkteil (20), wobei
- an dem Schwenkteil (20) zumindest ein Sperrsegment (22) mit daran angeordneten Formschlusselementen (23) angeordnet ist und
- an der Basis (10) zumindest ein Verriegelungselement (40) längsverschieblich entlang der Schwenkachse (30) gelagert ist, das aus einer Verriegelungsstellung, in der das Verriegelungselement (40) in Eingriff mit einem Formschlusselement (23) des Sperrsegmentes (22) steht, entgegen einer Federkraft in eine Freigabestellung bringbar ist, wobei das Verriegelungselement (40) zwei Freigabebereiche (41, 42) aufweist, mit denen eine erste und eine zweite Freigabestellung verwirklicht wird, der erste Freigabebereich (41) in Verschieberichtung beabstandet zu dem zweiten Freigabebereich (42) angeordnet ist und dem zweiten Freigabebereich (42) ein Sperrelement (50) zugeordnet ist, das das Verriegelungselement (40) in der zweiten Freigabestellung hält, wobei das Schwenkteil (20) entgegen einer Federkraft verschwenkbar an der Basis (10) gelagert ist und über zumindest eine in entgegengesetzte Richtungen wirkende Federkomponente (80) in einer Ausgangsstellung gehalten wird, wobei die Federkomponente (80) an einem Anschlag (288) angeordnet ist und das Widerlager (18) von dem Anschlag (288) weg außer Eingriff mit der Federkomponente (80) bringbar ist.

## Beschreibung

Die Erfindung betrifft ein Prothesenhandgelenk mit einer Basis zur Befestigung an einer proximalen Prothesenkomponente und einem um eine Schwenkachse an der Basis gelagerten Schwenkteil, mit zumindest einer Befestigungseinrichtung zur Festlegung einer distalen Prothesenkomponente oder Prothesenhand an dem Schwenkteil, wobei an dem Schwenkteil zumindest ein Sperrsegment mit daran angeordneten Formschlusselementen angeordnet und an der Basis zumindest ein Verriegelungselement längsverschieblich entlang der Schwenkachse gelagert ist, wobei das Verbindungselement aus einer Verriegelungsstellung, in der das Verriegelungselement in Eingriff mit einem Formschlusselement des Sperrsegmentes steht, entgegen einer Federkraft in eine Freigabestellung bringbar ist.

Prothesen ersetzen nicht vorhandene oder nicht mehr vorhandene Gliedmaßen. Prothesengelenke verbinden zwei Komponenten einer Protheseneinrichtung und ermöglichen eine Verschwenkung dieser Prothesenkomponenten gegeneinander. Aus dem Stand der Technik sind Prothesengelenke bekannt, bei denen eine Verschwenkung zweier Prothesenteile zueinander sperrbar ist. Je nach Aufbau der Prothesengelenke erfolgt die Sperrung bzw. Freigabe auf unterschiedliche Arten und Weisen. Ist beispielsweise zwischen einem Gelenkoberteil und einem Gelenkunterteil eine Widerstandseinrichtung, beispielsweise ein Hydraulikdämpfer, angeordnet, kann die Relativbewegung zwischen dem Oberteil und dem Unterteil durch eine Sperrung der Hydraulikleitung erreicht werden. Eine andere Art und Weise der Sperrung und Freigabe erfolgt bei einfachen, einachsigen Gelenken über mechanische Verriegelungen, mit denen die Gelenke in ihrer jeweiligen Winkelstellung zueinander fixiert werden. Wird die Verriegelung aufgelöst und befindet sich das distale Prothesenteil in einem entriegelten Zustand, ist es in der Regel frei beweglich. Bei Prothesenhandgelenken kann dies dazu führen, dass die Prothesenhand in Gravitationsrichtung absinkt oder herunterfällt.

Aus der DE 10 2006 020 777 B4 ist ein Prothesenhandgelenk mit einer Basis und einem der Basis zugeordneten Kopplungselement zur Befestigung an einem Körperglied oder einem Prothesenteil sowie mit einer mit der Basis um zumindest eine Achse verschwenkbar verbundenen Aufnahmeeinrichtung für eine Prothesenhand bekannt, wobei eine Verriegelungseinrichtung die Aufnahmeeinrichtung gegenüber der Basis blockiert. Ein Rückhalteelement ist elastisch gegen die Aufnahmeeinrichtung vorgespannt und beaufschlagt diese nach einer Entriegelung der Verriegelungseinrichtung mit einer Haltekraft. Die Aufnahmeeinrichtung weist Rastelemente auf, in die das Rückhaltelement in einer Verriegelungsstellung eingreift.

Aufgabe der vorliegenden Erfindung ist es, ein Prothesenhandgelenk bereitzustellen, das einen stabilen, robusten und kompakten Aufbau aufweist und eine erhöhte Flexibilität für den Anwender bereitstellt.

Gelöst wird diese Aufgabe durch ein Prothesenhandgelenk mit den Merkmalen des Hauptanspruches. Vorteilhafte Ausgestaltungen und Weiterbildungen der Erfindung sind in den Unteransprüchen, der Beschreibung sowie den Figuren offenbart.

Das Prothesenhandgelenk mit einer Basis zur Befestigung an einer proximalen Prothesenkomponente und einem Schwenkteil, das um eine Schwenkachse verschwenkbar an der Basis gelagert ist, mit zumindest einer Befestigungseinrichtung zur Festlegung einer distalen Prothesenkomponente oder Prothesenhand an dem Schwenkteil, wobei an dem Schwenkteil zumindest ein Sperrsegment mit daran angeordneten Formschlusselementen angeordnet und an der Basis zumindest ein Verriegelungselement längsverschieblich entlang der Schwenkachse gelagert ist, das aus einer Verriegelungsstellung, in der das Verriegelungselement in Eingriff mit einem Formschlusselement des Sperrsegmentes steht, entgegen einer Federkraft in eine Freigabestellung bringbar ist, sieht vor, dass das Verriegelungselement zwei Freigabebereiche aufweist, mit denen eine erste und eine zweite Freigabestellung des Verriegelungselementes verwirklicht wird, dass der erste Freigabebereich in Verschieberichtung beabstandet zu dem zweiten Freigabebereich angeordnet ist und dass dem zweiten Freigabebereich ein Sperrelement zugeordnet ist, das das Verriegelungselement in der zweiten Freigabestellung hält. Durch die Ausgestaltung des längsverschieblichen Verriegelungselementes mit zwei Freigabebereichen ist es möglich, das Prothesenhandgelenk in mehreren Freigabestellungen, ausgehend von der Verriegelungstellung, in der das Verriegelungselement in Eingriff mit einem der Formschlusselemente des Sperrsegmentes steht, in die Freigabestellung zu bringen. Die Federkraft, die gegen eine Verlagerung des Verriegelungselementes aus der Verriegelungsstellung in die Freigabestellung wirkt, wird durch ein Federelement aufgebracht, das mit dem Verriegelungselement in Eingriff steht. Das Verriegelungselement wird aus der Verriegelungsstellung entgegen der Federkraft in eine erste Freigabestellung entlang der Richtung der Längsorientierung der Schwenkachse verschoben, bis der erste Freigabebereich so positioniert ist, dass die erste Freigabestellung vorliegt. In der ersten Freigabestellung ist eine Verschwenkung des Schwenkteils relativ zu der Basis möglich, sodass sich beispielsweise die Prothesenhand aus einer Neutralstellung in eine angewinkelte Stellung verlagern lässt. Wird dann die Betätigungskraft entgegen der Federkraft reduziert oder aufgehoben, kehrt das Verriegelungselement in die Verriegelungsstellung zurück, sodass das Prothesenhandgelenk in der eingestellten Stellung verbleibt und formschlüssig darin verriegelt ist. Wird das Verriegelungselement in dessen zweite Freigabestellung bewegt, rastet ein Sperrelement mit dem Verriegelungselement ein oder tritt in Eingriff mit dem Verriegelungselement, sodass das Sperrelement das Verriegelungselement in der zweiten Freigabestellung hält. In der zweiten Freigabestellung liegt ebenfalls keine formschlüssige Verriegelung von dem Verriegelungselement mit dem Sperrsegment vor, sodass eine freie oder nahezu freie Beweglichkeit des Schwenkteils um die Schwenkachse gegeben ist. Zusätzlich wird durch das Sperrelement das Verriegelungselement in der zweiten Freigabestellung dauerhaft gehalten, sodass das Prothesenhandgelenk dauerhaft frei beweglich oder nahezu frei beweglich ist. Dies ist beispielsweise dann vorteilhaft, wenn eine Prothesenhand flach auf einer Tischoberfläche liegt, beim Händeschütteln oder beim Schulterklopfen, da dadurch eine natürlich wirkende Erscheinung des Handgelenkes erreicht wird.

In einer Ausgestaltung ist das Verriegelungselement aus der ersten Freigabestellung in die zweite Freigabestellung entgegen der Federkraft verschiebbar, sodass die beiden Freigabestellungen in der gleichen Verschieberichtung hintereinander angeordnet sind. Durch die Federkraft wird das Verriegelungselement aus der Freigabestellung zurück in die Verriegelungsstellung geschoben, sofern das Sperrelement nicht oder nicht mehr in Eingriff steht und die umgekehrte Bewegung in die Verriegelungsstellung blockiert. Dadurch ist es möglich, das Federelement zur Aufbringung einer Federkraft beispielsweise nur als Druckfeder auszugestalten, wenn das Verriegelungselement aus der Verriegelungsstellung entgegen der Federkraft in die Freigabestellung bewegt werden soll. Alternativ ist es möglich, dass die erste Freigabestellung in eine erste Verschieberichtung und die zweite Freigabestellung eine Verschiebung des Verriegelungselementes in die entgegengesetzte Richtung benötigt. Dazu sind entweder zwei Federelemente oder ein Federelement, das als Zugfederelement und Druckfederelement ausgebildet ist, vorgesehen.

In einer Ausgestaltung des Prothesenhandgelenkes ist die Federkraft gegen eine Verschiebung in die zweite Freigabestellung größer als die Federkraft gegen die Verschiebung in die erste Freigabestellung. Das Widerstandsverhalten für einen bestimmten, vorbestimmten Verschiebeweg des Verriegelungselementes von der Verriegelungsstellung bis in die erste Freigabestellung liegt auf einem ersten Niveau, während der sich daran anschließende Verschiebeweg oder der Verschiebeweg in die entgegengesetzte Richtung einen zweiten Widerstand bewirkt und eine entsprechend erhöhte Rückstellkraft erfordert, die größer als der erste Widerstand ist. Vorteilhafterweise ist der Übergang von dem ersten Widerstandsniveau zu dem zweiten Widerstandsniveau signifikant, sodass eine haptische Rückkopplung bei der Betätigung des Verriegelungselementes bereitgestellt wird.

Die Federkraft gegen eine Verschiebung in die zweite Freigabestellung ist in einer Ausgestaltung zu Beginn des zweiten Freigabebereiches signifikant größer, insbesondere 10 % größer als die Federkraft gegen die Verschiebung des ersten Freigabebereiches am Ende der ersten Freigabestellung.

Das Verriegelungselement ist in einer Ausgestaltung über zumindest ein Federelement gegen eine Verschiebung in eine Freigabestellung gegenüber der Basis abgestützt. Das Federelement kann zweistufig, mit einer abgestuften Federkennlinie und/oder mit einer progressiven Federkennlinie ausgebildet sein, um den haptischen Unterschied in der aufzubringenden Verschiebekraft entgegen der Federvorspannung bereitzustellen. Es können stattdessen oder auch als Ergänzung mehrere Federelemente in Verschieberichtung des Verriegelungselementes nacheinander in Eingriff anbringbar angeordnet sein, sodass nach Erreichen des ersten Freigabebereiches schlagartig bei Ineingrifftreten eines weiteren Federelementes oder weiterer Federelemente der erhöhte Widerstand gegen eine Verschiebung in den zweiten Federbereich vorhanden ist. Bei einer gegenläufigen Bewegung um das Verriegelungselement aus der Verriegelungsstellung in die erste oder zweite Freigabestellung zu bewegen, können in der jeweiligen Verschieberichtung unterschiedliche Federstärken oder Widerstände gegen eine Verschiebung bereitgestellt sein. Die Widerstände in der Federkraft haben bevorzugt mehr als 10 % Unterschied zueinander, um eine deutlich spürbare Unterscheidung zwischen den Freigabebereichen bereitzustellen. Die zweite Freigabestellung und der zweite Widerstand markieren die Stellung, in der eine Entriegelung stattgefunden hat und eine Verschwenkung um die Schwenkachse möglich ist, gegebenenfalls entgegen einer Rückstellkraft, die über Federelemente oder Elastomerelemente aufgebracht wird. Bei diesem zweiten, erhöhten Widerstandsniveau werden bei der Erreichung einer entsprechenden Position des Verriegelungselementes zumindest ein Sperrelement in Eingriff gebracht oder wirksam geschaltet, sodass eine Rückstellung durch das Federelement in die erste Freigabestellung oder die Verriegelungsstellung verhindert wird.

Dem Verriegelungselement können zwei einander in Verschieberichtung gegenüberliegende Betätigungselemente zugeordnet sein, sodass das Verriegelungselement in beide Verschieberichtungen längs der Längserstreckung der Schwenkachse leicht verschiebbar ist. Dadurch ist eine Betätigung in die erste und zweite Freigabestellung sowie zurück aus der zweiten, verriegelten Freigabestellung in die erste Freigabestellung oder Verriegelungsstellung einfach möglich.

In einer Ausgestaltung sind zwei parallel orientierte Verriegelungselemente beiderseits der Schwenkachse an der Basis gelagert. Durch die doppelte Ausgestaltung von Verriegelungselementen beiderseits der Schwenkachse ergibt sich eine zuverlässige, hohe Sperrfunktion aufgrund von zwei Sperrachsen mit einem vergleichsweise großen Hebelabstand zu der Schwenkachse.

Die Freigabebereiche des Verriegelungselementes sind in einer Ausgestaltung als Abflachungen oder Durchmesserverringerungen des Verriegelungselementes ausgebildet. In dem Bereich der Abflachungen oder Durchmesserverringerungen des Verriegelungselementes bzw. der Verriegelungselemente können die Sperrsegmente an dem Verriegelungselement vorbei verschwenken.

Um die leichte Verschwenkbarkeit und Funktionstüchtigkeit in allen Stellungen zu ermöglichen, ist in einer Ausgestaltung das Sperrsegment kreissegmentförmig ausgebildet und an dem Schwenkteil befestigt oder angeformt.

Es können auch mehrere Sperrsegmente voneinander beabstandet, insbesondere entlang der Längserstreckung der Schwenkachse zueinander beabstandet an dem Schwenkteil befestigt oder ausgebildet sein, um eine zuverlässige Verriegelung und gleichmäßige Kraftaufnahme und Kraftübertragung zu gewährleisten.

In einer Ausgestaltung ist das Sperrelement in Richtung auf das Verriegelungselement federbelastet, sodass automatisch nach Erreichen der zweiten Freigabestellung oder des zweiten Freigabebereiches das Sperrelement einrastet oder mit dem Verriegelungselement in Eingriff tritt, wobei das Verriegelungselement in einer Ausgestaltung eine Formschlusskomponente aufweist, mit der das Sperrelement formschlüssig in Eingriff bringbar ist.

Das Schwenkteil kann entgegen einer Federkraft verschwenkbar an der Basis gelagert sein, um auch in einer Freigabestellung eine gewisse Rückstellkraft in eine Ausgangsposition bereitstellen zu können.

Das Schwenkteil kann über zumindest eine in entgegengesetzte Richtungen wirkende Federkomponente, insbesondere über zwei entgegengesetzt wirkende Federkomponenten in einer Ausgangsstellung gehalten sein bzw. entgegengesetzt wirkende Federkräfte ausgesetzt sein, die das Schwenkteil in Richtung auf eine Ausgangsstellung bewegen oder eine Kraft in Richtung auf die Ausgangsstellung ausüben.

Die Federkomponenten zur Aufbringung einer Federkraft in Richtung auf eine Ausgangsstellung können in Ausnehmungen an der Basis oder dem Schwenkteil, insbesondere dem Sperrsegment angeordnet sein und stützen sich an Widerlagern an dem jeweils gegenüberliegenden Element ab. Bei einer Anordnung der Federkomponenten in Ausnehmungen an der Basis ist das Widerlager an dem Schwenkteil, insbesondere Sperrsegment angeordnet, bei einer Ausgestaltung der Ausnehmungen in dem dem Schwenkteil, insbesondere in einem Sperrsegment, ist das Widerlager an der Basis angeordnet oder ausgebildet, woran sich die jeweilige Federkomponente abstützt. Das jeweilige Widerlager kann als Buchse ausgebildet sein, die an dem Schwenkteil, insbesondere Sperrsegment oder an der Basis festgelegt ist.

In einer Weiterbildung ist der Federkomponente ein Anschlag zugeordnet, an dem die Federkomponente anliegt, insbesondere in einem vorgespannten Zustand. Das Widerlager liegt in einer vorbestimmten Stellung, beispielsweise in einer Neutralstellung, an der Federkomponente an und ist von der Federkomponente weg von dem Anschlag verlagerbar an dem Schwenkteil oder der Basis angeordnet. Dadurch ist es möglich, die Auslösekraft zu Verschwenkung aus der Neutralstellung einzustellen, insbesondere zu erhöhen, um eine stabile Neutralstellung zu erreichen.

Nachfolgend werden Ausführungsbeispiele der Erfindung anhand der Figuren näher erläutert. Es zeigen:
Figur 1 - eine Explosionsdarstellung eines Prothesenhandgelenkes;
Figur 2 - eine schematische Ansicht in einem Vertikalschnitt in der Sperrstellung;
Figur 3 - eine Darstellung gemäß Figur 2 in der ersten Freigabestellung;
Figur 4 - eine Darstellung gemäß Figur 2 in der zweiten Freigabestellung;
Figur 5 - eine Variante der Figur 1;
Figur 6 - unterschiedliche Federkennlinien;
Figur 7 - eine Seitenansicht gemäß Figur 5;
Figuren 8-10 - unterschiedliche Stellungen des Prothesenhandgelenkes; sowie
Figuren 11 und 12 - Varianten des Sperrmechanismus.

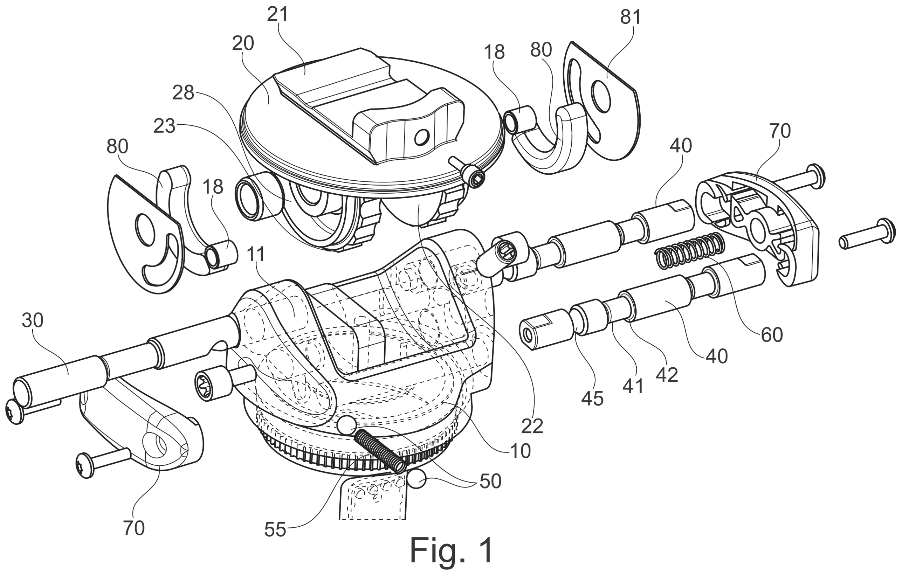

In der Figur 1 ist eine perspektivische Explosionsdarstellung eines Prothesenhandgelenkes mit einer Basis 10 gezeigt, an der zwischen zwei Lagerböcken 11 ein Schwenkteil 20 um eine Schwenkachse 30 verschwenkbar gelagert ist. Die beiden Lagerböcke 11 erstrecken sich in distaler Richtung von einer Grundplatte der Basis 10. In proximaler Richtung von der Grundplatte ist in dem Ausführungsbeispiel ein Drehlager angeordnet, um das das Prothesenhandgelenk drehbar an einem Unterarmschaft oder einem Unterarmrohr befestigt werden kann. Die Basis 10 ist insbesondere einstückig oder einteilig ausgebildet. Zwischen den beiden Lagerböcken 11 ist ein Freiraum ausgebildet, in dem das Schwenkteil 20 angeordnet ist. Das Schwenkteil 20 weist an seiner distalen Oberseite eine Befestigungseinrichtung 21 zur Festlegung einer nicht dargestellten distalen Prothesenkomponente, insbesondere einer Prothesenhand auf. Die Befestigungseinrichtung 21 in dem dargestellten Ausführungsbeispiel ist eine Schwalbenschwanzführung, auf die eine entsprechende Schwalbenschwanznut des zu befestigenden Bauteils aufgesteckt werden kann. Alternativ ist die Befestigungseinrichtung 21 als eine Überwurfmutter ausgebildet, wie später erläutert werden wird. An der der Befestigungseinrichtung 21 gegenüberliegenden Unterseite sind zwei Sperrsegmente 22 angeordnet, die als scheibenförmige, kreissegmentartige Vorsprünge ausgebildet und in dem montierten Zustand zwischen den Lagerböcken 11 angeordnet sind. An dem Außenumfang der Sperrsegmente 22 sind Formschlusselemente 23 in Gestalt von Vorsprüngen und Einkerbungen ausgebildet, die Formschlusselemente 23 sind insbesondere gleichmäßig um den Umfang verteilt. Die Formschlusselemente 23 an den beiden axial beabstandet zueinander angeordneten Sperrsegmente 22 sind zueinander ausgerichtet, sodass eine stufenweise Verstellung und Fixierung bzw. Freigabe in der jeweils gewünschten Position stattfinden kann.

An der Basis 10 sind in den Lagerböcken 11 zwei Verriegelungselemente 40 in Gestalt von längsverschieblichen Stangen oder Bolzen gelagert. Die beiden Verriegelungselemente 40 sind parallel zueinander ausgerichtet und verlaufen parallel zu der Längserstreckung der Schwenkachse 30. An den beiden Stirnseiten der Verriegelungselemente 40 sind Betätigungselemente 70 in Gestalt von Druckplatten angeordnet und über Schrauben mit den Verriegelungselementen 40 verbunden. Die Verriegelungselemente 40 stützen sich über ein Federelement 60 oder mehrere Federelemente gegenüber der Basis 10 ab, sodass eine Verlagerung und Verschiebung der Verriegelungselemente 40 innerhalb von Kanälen, die in der Basis 10 ausgebildet sind, entgegen einer Federkraft oder einer Vorspannung erfolgt.

Die Verriegelungselemente 40 in der dargestellten Ausführungsform sind stabförmig ausgebildet und weisen einen im Wesentlichen runden Querschnitt auf. Entlang der Längserstreckung der Verriegelungselemente 40 sind Abflachungen oder Durchmesserverringerungen ausgebildet, die in den jeweiligen Stellungen in Ausrichtung zu den jeweiligen Sperrsegmenten 22 gebracht werden können. Die Funktionsweise wird später erläutert werden. Die Bereiche mit größerem Durchmesser stehen in einer Sperrstellung in Eingriff mit den Formschlusselementen 23 an den Sperrsegmenten 22. Werden die Verriegelungselemente 40 verschoben, werden die Bereiche mit einem größeren Durchmesser außer Eingriff mit den Formschlusselementen 23 gebracht, sodass die Bereiche mit dem geringeren Durchmesser oder mit den Materialausnehmungen den Formschlusselementen 23 gegenüber liegen. In dem dargestellten Ausführungsbeispiel sind für jedes Sperrsegment 22 zwei Freigabebereiche 41, 42 an dem jeweiligen Verriegelungselement 40 ausgebildet. Die beiden Freigabebereiche 41, 42 teilen sich in einen ersten Freigabebereich 41 und einem zweiten Freigabebereich 42 auf. Der erste Freigabebereich 41 kommt in Überdeckung mit den Formschlusselementen 23 des jeweiligen Sperrsegmentes 22 in einer ersten Freigabestellung. Direkt daran schließt sich der zweite Freigabebereich 42 in axialer Verschieberichtung hinter dem ersten Freigabebereich 41 an. In beiden Freigabestellungen 41, 42 blockieren die Verriegelungselemente 40 die Verschwenkung des Schwenkelementes 20 um die Schwenkachse 30 nicht. Zusätzlich ist an den Verriegelungselementen 40 eine Formschlusskomponente 45 in Gestalt einer umlaufenden Nut ausgebildet, die so angeordnet ist, dass ein Sperrelement 50 in der zweiten Freigabestellung und nur in der zweiten Freigabestellung in Eingriff mit der Formschlusskomponente 45 treten kann. Das Sperrelement 50 ist in dem dargestellten Ausführungsbeispiel als eine Kugel ausgebildet, die über eine Feder 55 in Richtung auf das jeweilige Verriegelungselement 40 vorgespannt innerhalb der Basis 10 in einer Bohrung gelagert ist. Die Formschlusskomponente 45 ist beabstandet zu den Freigabebereichen 41, 42 und getrennt davon an dem jeweiligen Verriegelungselement 40 ausgebildet.

In dem zusammengebauten Zustand befinden sich die Sperrelemente 50 unter Vorspannung durch das Federelement 55 in Anlage an den Verriegelungselementen 40. Da die beiden im Wesentlichen parallel zueinander orientierten Verriegelungselemente 40 beiderseits gleichbeabstandet zu der Projektion der Schwenkachse 30 liegen und die Außendurchmesser außerhalb der Freigabebereiche 41, 42 und der Formschlusskomponente 45 mit den Vorsprüngen der Formschlusselemente 23 in Eingriff treten, ist eine Verschwenkbewegung des Schwenkelementes 20 relativ zu der Basis 10 gesperrt. Die vergrößerten Durchmesserbereiche oder Sperrbereiche der Verriegelungselemente 40 lassen sich entlang der Formschlusselemente 23 bzw. der Ausnehmungen an dem Außenumfang der Sperrsegmente 22 in Längsrichtung entgegen der Federkraft durch die Feder 60 verschieben. Somit befindet sich das Prothesenhandgelenk in einer Ausgangsstellung in einer Sperrstellung oder Verriegelungsstellung und wird durch Druck auf ein Betätigungselement 70 gegen die Federkraft 60 entsperrt. Dazu werden die Verriegelungselemente 40 gegen die Federkraft 60 aus der Sperrstellung oder Verriegelungsstellung in eine Freigabestellung verlagert. In der ersten Freigabestellung ist es möglich, dass das Schwenkteil 20 relativ zu der Basis 10 um die Schwenkachse 30 verschwenkt. Die Schwenkachse 30 ist über eine Madenschraube innerhalb der Lagerböcke 11 gegen eine Verdrehung und gegen eine Axialbewegung gesichert. Die Schwenkachse 30 ist in einer Alternative zweiteilig ausgeführt, wobei jede Halbachse an jeder Seite durch eine Madenschraube gesichert wird. Das Schwenkteil 20 ist auf Lagerbuchsen an der Schwenkachse 30 gelagert.

An den Außenseiten der Sperrsegmente 22 sind Ausnehmungen 28 angeordnet, die jeweils nach außen in Richtung auf die Lagerböcke 11 geöffnet sind. Die Ausnehmungen 28 sind kreissegmentförmig, beispielsweise halbkreisförmig ausgebildet und nehmen jeweils eine Federkomponente 80 auf. In dem dargestellten Ausführungsbeispiel sind die Federkomponenten 80 einstückig ausgebildet und als Elastomerelemente korrespondierend zu der Form der Ausnehmung 28 geformt. An auf gegenüberliegenden Seiten der Schwenkachse 30 angeordneten Widerlagern 18, die an der Basis 10 an den Lagerböcken 11 über Schrauben oder Achsen fixiert sind, stützt sich die jeweilige Federkomponente 80 gegenüber der Basis 10 ab. Bei einer Verschwenkung in die eine Richtung ausgehend von einer Ausgangsstellung wird eine Federkomponente 80 komprimiert, bei einer Verschwenkung ausgehend von der Ausgangsstellung in die andere Richtung die jeweils andere. Somit ist immer nur eine Federkomponente 80 bei einer Verschwenkung aus der Ausgangsstellung relativ zu der Ausgangsstellung komprimiert. Die Federkomponenten 80 können so ausgestaltet sein, dass eine Grundspannung vorhanden ist, sodass auch in einer Freigabestellung eine Verschwenkung um die Schwenkachse 30 nur nach Überwindung einer vorgegebenen, vorzugsweise einstellbaren Federkraft möglich ist.

Alternativ zu der dargestellten Ausführungsform kann die entsprechende Ausnehmung für die Federkomponente 80 auch in dem Lagerbock 11 der Basis ausgebildet sein, das Widerlager ist dann an dem jeweiligen Sperrsegment 22 angeordnet oder ausgebildet. Nach außen werden die Ausnehmungen 28 über Abdeckscheiben 81, insbesondere Edelstahlscheiben abgedeckt. Innerhalb der Abdeckscheiben 81 sind Kulissen ausgebildet, sodass die Achsen oder Stifte, die von den Lagerböcken 11 zu den Buchsen als Widerlager 18 führen, eine Verschwenkung der Abdeckscheiben 81 mit dem Schwenkelement 20 nicht behindern.

Durch die Federkomponenten 80 ist es möglich, eine elastische Ausgangsstellung oder Null-Stellung zu erreichen. Die gegensinnige Anordnung der Federkomponenten 80 ermöglicht eine gleichmäßige oder unterschiedliche Gegenkraft gegen eine Verschwenkung aus der Ausgangsstellung. Die Abdeckscheiben 81 dienen neben einem Schutz vor Verschmutzung auch der Begrenzung des Volumens der Ausnehmungen 28, sodass bei einer Verformung der als Elastomerkomponenten ausgebildeten Federkomponenten 80 diese nicht aus den Ausnehmungen 28 herausgedrückt werden. Darüber hinaus dienen die Abdeckscheiben 81 zum Spielausgleich. Alternativ zu einer Ausgestaltung der Federkomponenten 80 als Elastomerelemente können diese auch als Wendelfedern oder Schraubenfedern ausgebildet sein. Die Lagerung der Elastomerkomponenten, insbesondere der halbmondförmigen Elastomerelemente in korrespondierend geformten Aufnahmen kann auch ohne die Verriegelungselemente 40 mit den Freigabebereichen 41, 42 und der Feststellung oder Fixierung in der zweiten Freigabestellung eingesetzt werden und ist ein weiterer Aspekt der Erfindung, der auch unabhängig und eigenständig weiterverfolgt werden kann.

Die Formschlusselemente 23 sind in dem Ausführungsbeispiel teilkreisförmig ausgebildet und definieren Raststellungen, in denen eine Festlegung der Orientierung des Schwenkteils 20 relativ zu der Basis 10 erfolgen kann. Die Verriegelungselemente 40 sind vorteilhafterweise in den Eingriffsbereichen im Querschnitt rund ausgebildet, was eine verbesserte Flächenpressung in der Sperrstellung bewirkt. In der Figur 1 zu erkennen, dass sowohl an den Sperrsegmenten 23 als auch an den Verriegelungselementen 40 Einführschrägen ausgebildet sind, sodass durch die Federkraft der Feder 60 die Verriegelungselemente 40 oder Sperrstifte in die jeweilige Ausnehmung oder das Formschlusselement 23 an dem Außenumfang der Sperrsegmente 22 eingreifen können. Die Feder 60 kann zweistufig, progressiv, kegelig oder mehrteilig ausgebildet sein.

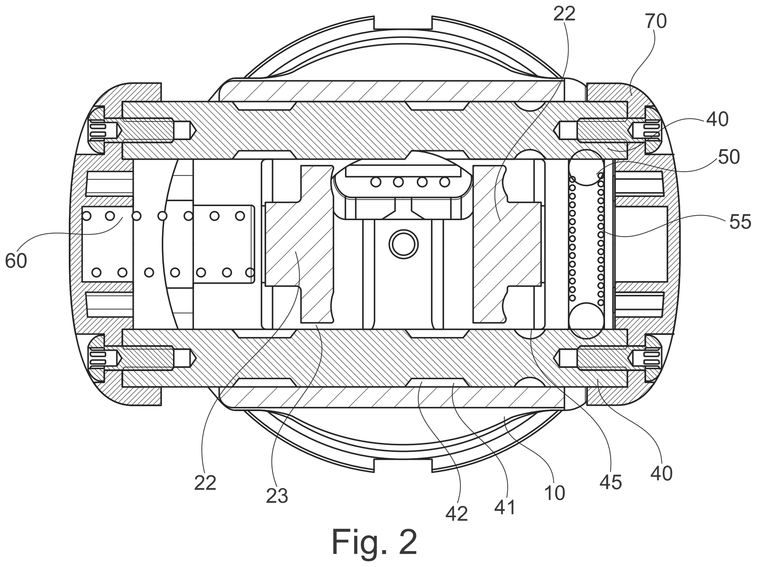

In der Figur 2 ist eine Draufsicht durch einen Schnitt in der Ebene der Verriegelungselemente 40 in der verriegelten Ausgangsstellung gezeigt. Die stabförmigen Verriegelungselemente 40 sind parallel zueinander angeordnet und beiderseits der Sperrsegmente 22 parallel zu der nicht dargestellten Schwenkachse orientiert an der Basis 10 verschieblich gelagert. Das Federelement 60 ist vorgespannt an der Basis 10 und an dem Betätigungselement 70 gelagert und übt eine Vorspannkraft in Richtung auf die dargestellte Sperrstellung aus. Die Verriegelungselemente 40 stehen in Eingriff mit den Formschlusselementen 23 der Sperrsegmente und verhindern eine Verschwenkung um die Schwenkachse. Die Sperrelemente 50 werden über die Feder 55 in Richtung auf die Verriegelungselemente 40 vorgespannt und liegen an der Außenseite der Verriegelungselemente 40 an und lassen eine Verschiebung der Verriegelungselemente 40 zu.

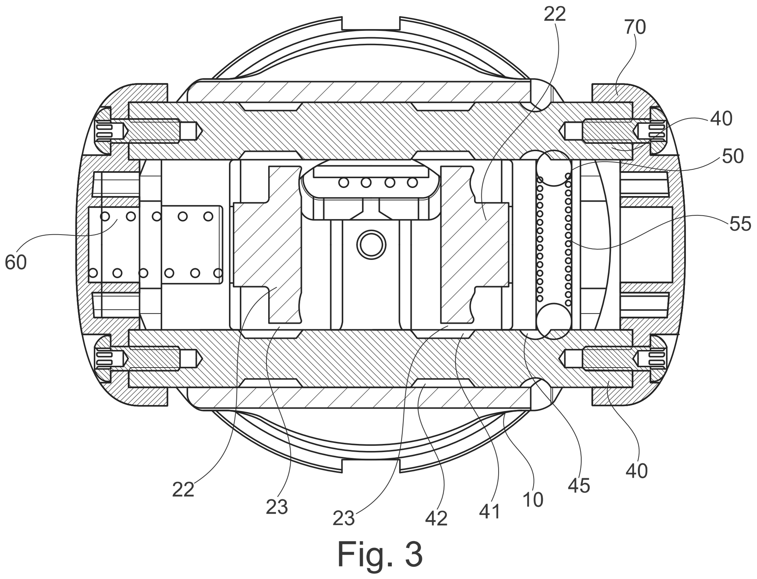

In der Figur 3 ist die Stellung der Verriegelungselemente 40 nach einer Verlagerung nach rechts in eine erste Freigabestellung dargestellt. Die Formschlusselemente 23 der Sperrsegmente 22 befinden sich nunmehr gegenüber dem ersten Freigabebereich 41, der als Bereich einer umlaufenden Nut ausgebildet ist. Die vorher vorhandene formschlüssige Verriegelung zwischen den Verriegelungselementen 40 und den Sperrsegmenten 22 an vier einander gegenüberliegenden Stellen ist nunmehr aufgehoben, sodass sich das Schwenkteil 20 entgegen der Federkraft durch die Federkomponenten 80 um die Schwenkachse 30 verschwenken lässt. Die Sperrelemente 50 in Gestalt der Kugeln rollen weiterhin an der Außenseite der Verriegelungselemente 40 ab und befinden sich noch nicht in Eingriff mit der Formschlusskomponente 45 des Verriegelungselementes 40. Wird in dieser Stellung keine weitere Druckkraft von der linken Seite entgegen der Feder 60 aufgebracht, bewegen sich die Verriegelungselemente 40 wieder zurück in die Ausgangsstellung gemäß der Figur 2. Die dargestellte erste Freigabestellung dient dazu, eine willentliche Verschwenkung des Schwenkteils 20 gegenüber den Federkomponenten 80 durchzuführen und dann die Prothesenhand in dieser Position zu fixieren, indem automatisch eine Rückbewegung in die Verrieglungsstellung erfolgt, wenn von dem Nutzer keine Druckkraft mehr aufgebracht wird.

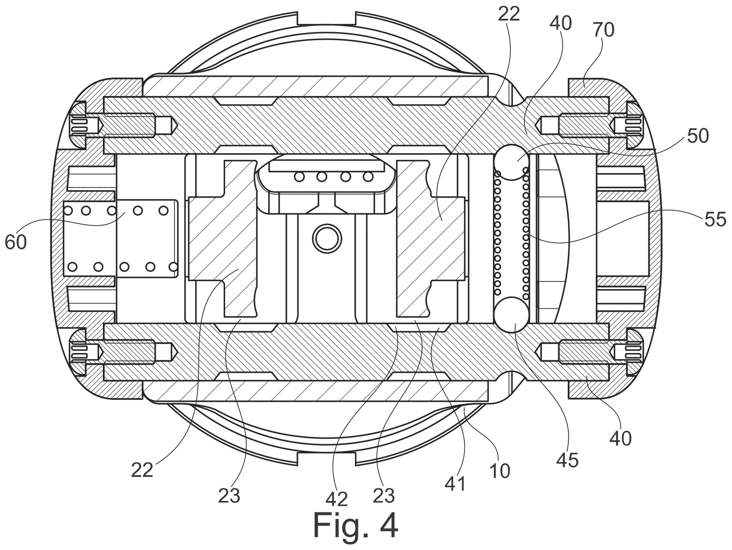

Ist eine dauerhafte Freigabe und Verschwenkbarkeit gegen die Federkomponenten 80 gewünscht, wird eine weitere Verschiebung der Verriegelungselemente 40 nach rechts durchgeführt, bis die Sperrelemente 50 in die Formschlusskomponenten 45 einrasten. Dies ist in der Figur 4 dargestellt. Die Federkraft der Feder 55 ist so eingestellt, dass auch nach Wegfall einer Verschiebekraft gegen die Feder 60 die zweite Freigabestellung gemäß der Figur 4 beibehalten wird. In dieser zweiten Freigabestellung befinden sich die Formschlusselemente 23 der Sperrsegmente 22 weiterhin außer Eingriff mit den Verriegelungselementen 40 und stehen den zweiten Freigabebereichen 42 gegenüber. Der zweite Freigabebereich 42 ist so positioniert und gewählt, dass das Sperrelement 50 in die Formschlusskomponente 45 einrastet. Zur Überwindung dieser dauerhaften Freigabestellung wird eine Betätigungskraft nach links wirkend in Richtung der Vorspannkraft der Feder 60 ausgeübt, wodurch die Sperrelemente 50 nach innen gegen die Feder 55 verlagert werden und eine Verschiebung der Verriegelungselemente 40 bewirkt wird.

Um den Übergang von dem ersten Freigabebereich zum zweiten Freigabebereich für den Nutzer spürbar zu machen, können mehrere Federelemente 60 vorgesehen sein, die nacheinander in Eingriff treten, sodass sich eine schlagartige und spürbare Vergrößerung der Widerstandskraft gegen eine Verschiebung aus dem ersten Freigabebereich in den zweiten Freigabebereich einstellt.

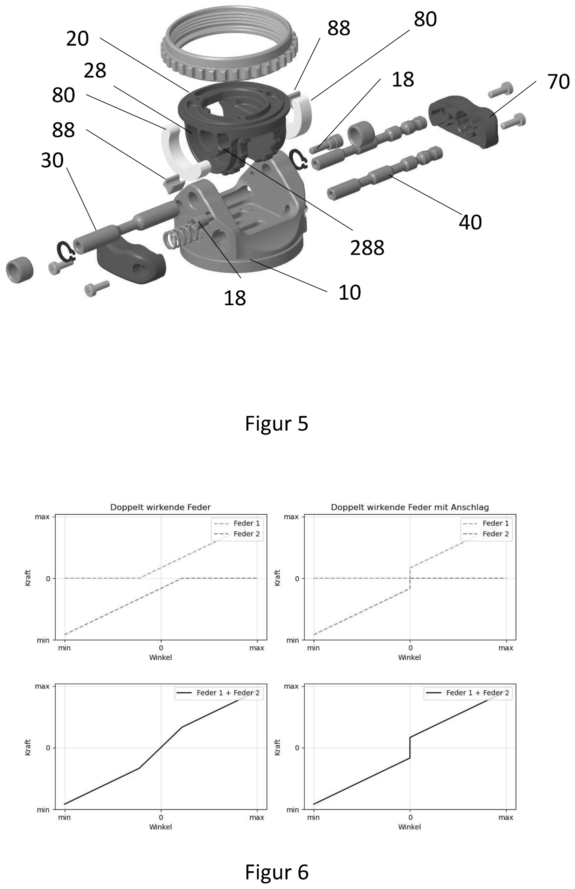

In der Figur 5 ist eine weitere Ausführungsform des Prothesenhandgelenkes gezeigt, die im Wesentlichen der Ausführungsform der Figur 1 entspricht. Gleiche Bezugszeichen bezeichnen die gleichen Bauteile. Aus Gründen der Übersichtlichkeit sind nicht alle Bezugszeichen der Figur 1 auch in der Figur 5 eingetragen. Der wesentliche Unterschied zwischen den Ausführungsformen der Figur 1 und der Figur 5 besteht darin, dass an dem Schwenkteil 20 in dem Bereich der Sperrsegmente die Ausnehmungen 28, in die die gebogenen Federkomponenten 80 eingesetzt sind, jeweils mit einem Anschlag 288 versehen sind, der in der dargestellten Ausführungsform als eine Verengung ausgebildet ist. Der Anschlag 288 kann auch als eine separate Komponente ausgebildet sein. Der Anschlag 288 kann an unterschiedlichen Stellen an dem Sperrsegment in der Ausnehmung 28 oder an der Ausnehmung 28 angeordnet sein, um die Position des Anschlages 288 einstellen und variieren zu können. Die Federkomponente 80 stützt sich über einen Mitnehmer 88 an dem jeweiligen Widerlager 18, dass an der Basis 10 befestigt ist, ab. Die Widerlager 18 gehen bei einer Verschwenkung durch die Verengung des Anschlages 288 hindurch oder kommen nicht in Kontakt mit dem Anschlag 288, wenn die Verschwenkung des Schwenkteils 20 um die Schwenkachse 30 ausgeführt wird. Je nach Verschwenkrichtung und Verschwenkstellung werden die jeweiligen Widerlager 18 in Eingriff mit dem Mitnehmer 88 gebracht oder bewegen sich frei ohne Kontakt zu dem Mitnehmer 88, wenn die Widerlager 18 sich zwischen den Vorsprüngen des Anschlages 288 oder entfernt davon auf der dem Mitnehmer 88 abgewandten Seite befinden. Die Funktionsweise wird weiter unten erläutert werden.

In der Figur 6 sind die Federkennlinien der einzelnen Federkomponenten in der oberen Darstellung gezeigt, in der jeweils unteren Darstellung ist die kombinierte oder resultierende Federkennlinie der gesamten Federanordnung der Federkomponenten 80 dargestellt. Die linke Darstellung zeigt die Federkennlinie der doppelt wirkenden Federkomponenten 80 gemäß der Figur 1, die rechte Darstellung die Ausgestaltung mit den Mitnehmern 88 und dem Anschlag 288 gemäß Figur 5. Bei beiden Ausführungsformen befinden sich zwischen der Basis 10 und dem Schwenkteil 20 zwei einander entgegengerichtet wirksame Federkomponenten 80, die aufgrund der Ausgestaltung als Elastomerkomponenten mit einer Festkörperdämpfung auch eine dämpfende Funktion ausüben können. Die beiden Widerlager 18 für die einander gegenüberliegenden Federkomponenten 80, die beispielsweise als Lagerschrauben ausgebildet sein können, sind mit der Basis 10 verbunden und ragen in die Ausnehmungen 28 hinein, in denen die Federkomponenten 80 und auch die Mitnehmer 88 angeordnet sind. Die Widerlager 18 übertragen die Federkraft von den Federkomponenten auf das Schwenkteil 20. Beide Federkomponenten 80 sind als Druckfedern ausgebildet, zumindest in dem dargestellten Ausführungsbeispielen, und sind in der Neutralposition, in der keine Flexion oder Extension des Schwenkteils 20 vorgenommen ist, vorgespannt. Beide Federkomponenten 80 wirken in entgegengesetzte Richtungen. Da die Federkräfte der Federkomponenten 80 in entgegengesetzte Richtungen wirken, ergibt sich die resultierende Kraft aus der Differenz der beiden Federkräfte. Ein exemplarischer Verlauf der entgegengesetzt wirkende Federkomponenten 80 der Figur 1 ist in der linken oberen Darstellung der Figur 6 gezeigt. Wenn beide Federkomponenten 80 bis zur vollständigen Entspannung Kraft auf die Basis 10 in gleicher Größe übertragen, heben sich dadurch in der Neutralposition die Federkräfte vollständig auf oder, umgekehrt ausgedrückt, wenn die Federkräfte sich vollständig aufheben, befindet sich das Schwenkteil 20 in der Ruhestellung oder Neutralstellung. Die resultierende Federkennlinie aus einer solchen Anordnung ist in der unteren linken Darstellung der Figur 6 gezeigt. Eine signifikante Rückstellkraft kann somit erst ab einem gewissen Flexionswinkel oder Extensionswinkel erreicht werden, wodurch sich ein vergleichsweise hohes Spiel des Prothesenhandgelenkes um die Neutralstellung ergibt.

Um eine höhere Stabilität des Schwenkteils 20 relativ zu der Basis 10 zu erreichen, ist es möglich, die Kraft einer Federkomponente 80 erst ab einem definierten Winkel in dem Schwenkteil 20 abzuleiten und damit zu neutralisieren. Dadurch kann bereits bei geringen Auslenkungen aus der Neutralposition eine deutlich höhere Rückstellkraft bereitgestellt werden. Es bedarf somit einer höheren Kraft zur Auslenkung des Schwenkteils 20 aus der Neutralstellung bzw. der Prothesenhand relativ zu dem Schaft. Dies wird dadurch erreicht, dass die vorgespannte Federkomponente 80 über den Mitnehmer 88 mit einer hohen Vorspannung in der jeweiligen Wirkrichtung versehen ist und das Widerlager 18 in der entgegengesetzten Richtung außer Kontakt mit dem Mitnehmer 88 gebracht werden kann. Die Anordnung eines Mitnehmers 88 hat einige Vorteile, über den separaten Mitnehmer 88 ist es beispielsweise möglich, dass unterschiedliche Materialien zum Einsatz kommen. Der Mitnehmer 88 kann wesentlich steifer oder starrer als die Federkomponente 80 ausgebildet sein, sodass eine verbesserte und präzisere Kraftübertragung von der Basis 10 über das Widerlager 18 auf die Federkomponente 80 stattfinden kann. Sowohl das Widerlager 18 als auch der Mitnehmer 88 können aus Metall oder einem harten Kunststoff bestehen, sodass sich bei dem Kontakt des Widerlagers 18 mit dem Mitnehmer 88 keine Verformung einstellt. Darüber hinaus ist es möglich, über den austauschbaren Mitnehmer 88 die Federvorspannung der Federkomponenten 80 einzustellen. Je größer der Mitnehmer 88 ist, desto höher ist die Kompression und damit die Vorspannung der Federkomponente 80. Grundsätzlich ist es aber auch möglich, den Mitnehmer 88 als Teil der Federkomponente 80 auszubilden oder die Federkomponente 80 so auszugestalten, dass das Widerlager 18 außer Eingriff mit der Federkomponente 80 gebracht werden kann, indem die Federkomponente 80 an dem Anschlag 288 anliegt.

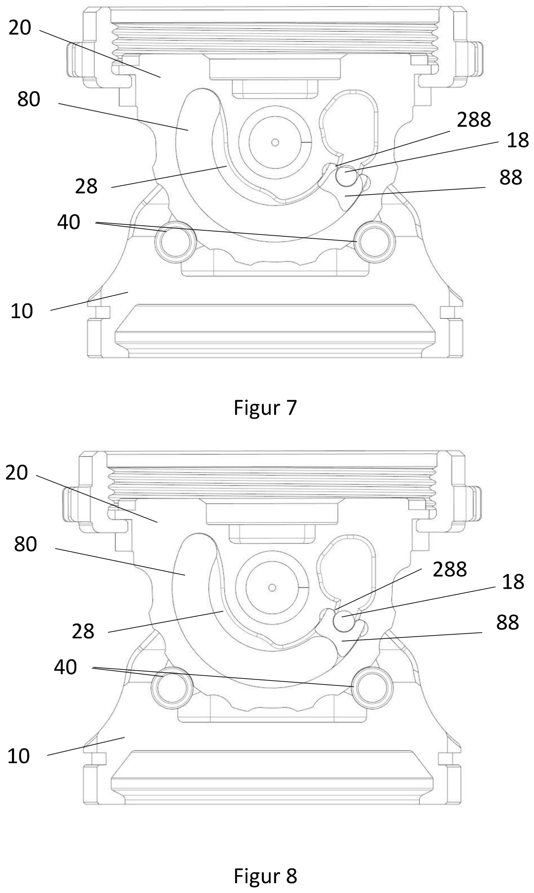

In der Figur 7 ist eine schematische Seitenansicht des Prothesenhandgelenkes gemäß Figur 5 gezeigt, bevor die Federkomponente 80 komprimiert in der Ausnehmung 28 eingeführt ist. Die gebogenen Federkomponente 80 ist in der gleichartig oder ähnlich gebogenen Ausnehmung 28 eingelegt und reicht bis zu der Verjüngung, die den Anschlag 288 ausbildet. Die Verjüngung innerhalb der Ausnehmung 28 ist so ausgebildet, dass das Widerlager 18 an der Basis 10 durch einen Kanal oder Freiraum hindurch gehen und in das rechte, freie Ende der Ausnehmung 28 eintreten kann. In der dargestellten Neutralposition befindet sich die Federkomponente 80 in einem nicht komprimierten Zustand, der Mitnehmer 88 ist in der Position an dem Anschlag 288 befindlich positioniert, und das Widerlager 18 liegt in einer Ausnehmung in dem Mitnehmer 88 an.

In der Figur 8 ist, das Prothesenhandgelenk fertig montiert. Beide Federkomponenten 80 sind auf einander gegenüberliegenden Seiten angeordnet und innerhalb der jeweiligen Ausnehmung 28 eingeführt. Der Mitnehmer 88 befindet sich zwischen dem Anschlag 288 und dem rechten Ende der Federkomponente 80. Das Widerlager 18 ragt in die Ausnehmung 28 hinein und kann in beide Richtungen bewegt werden, je nachdem, wie das Schwenkteil 20 relativ zu der Basis 10 um die Schwenkachse verschwenkt wird. In dieser Ausgangsposition sind aufgrund der Federvorspannung der Federkomponenten 80 vergleichsweise hohe Kräfte notwendig, um das Schwenkteil 20 entgegen dem Uhrzeigersinn zu verlagern. Eine Verschwenkung in Uhrzeigersinn ist durch die dargestellte Federkomponente 80 nicht behindert, dies wird durch die Federkomponente auf der gegenüberliegenden Seite an dem anderen Sperrsegment bewirkt.

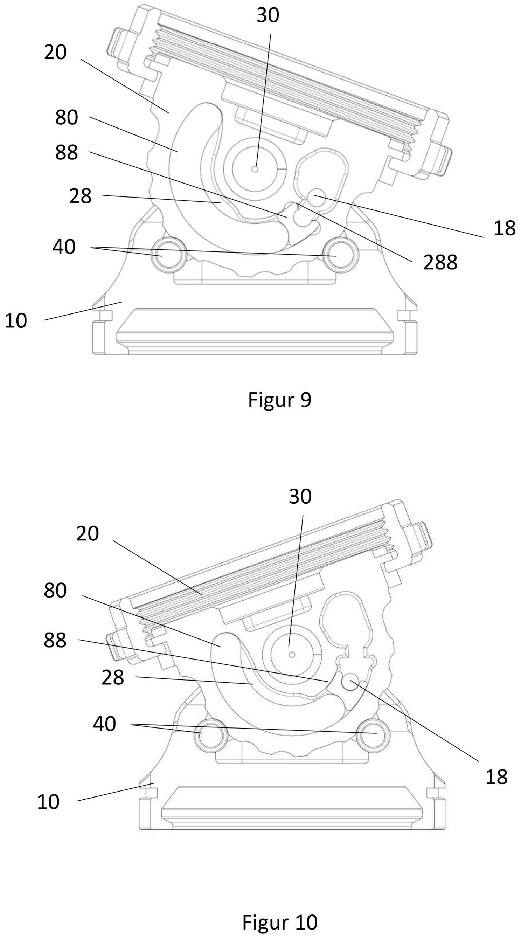

In der Figur 9 ist eine Verschwenkung des Schwenkteils 20 in Uhrzeigerrichtung um die Schwenkachse 30 dargestellt. Das Widerlager 18 ist außer Eingriff mit dem Mitnehmer 88 gebracht, der weiterhin gegen den Anschlag 288 durch die vorgespannte Federkomponente 80 gedrückt wird. Wird, wie in der Figur 10 dargestellt, das Schwenkteil 20 gegen den Uhrzeigersinn um die Schwenkachse 30 verschwenkt, tritt ab der Neutralstellung, die in der Figur 8 dargestellt ist, das Widerlager 18 in Eingriff mit dem Mitnehmer 88 und eine Widerstandskraft gegen die Verschwenkung wird durch die Federkomponente 80 bereitgestellt. Je nach Höhe der Vorspannkraft ist es möglich, die Auslösekraft für eine Verschwenkung in die eine oder andere Richtung einzustellen. Ebenso ist es möglich, unterschiedliche Auslösekräfte einzustellen, also für eine Extension eine höhere Auslösekraft als für eine Flexion oder umgekehrt bereitzustellen.

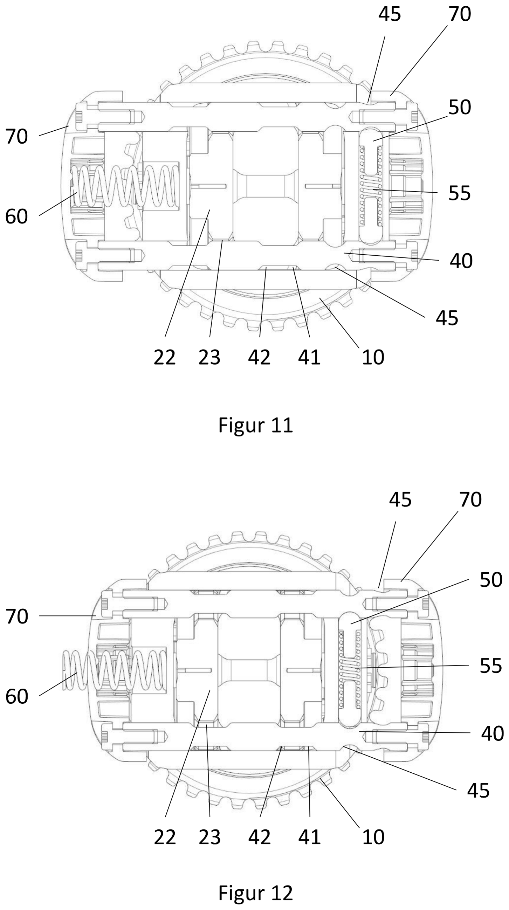

In der Figur 11 und 12 sind Schnittdarstellungen einer Variante des Sperrmechanismus gemäß der Figuren 2 bis 4 gezeigt. In der oberen Darstellung befindet sich das Betätigungselement in der Stellung gemäß der Figur 2, die Sperrsegmente 22 befinden sich mit ihren Formschlusselementen 23 beide in Eingriff mit den Verriegelungselementen 40. Zusätzlich befinden sich die Sperrelemente 50, die durch die Druckfeder 55 nach außen gedrückt werden, in Ausnehmungen oder Vertiefungen in Gestalt von Nuten als Formschlusskomponente der Verriegelungselemente 40, so dass der Sperrmechanismus mit den Betätigungselementen 70 in der Regelungsposition gehalten wird. In der Figur 12 ist die vollständig freigegebene und geöffnete Position, wie sie auch in der Figur 4 dargestellt ist, dargestellt. Die Sperrelemente 50, die als Stifte mit kugelförmigen Köpfen ausgebildet sein können, greifen in die Formschlusskomponente 45 an dem Außenumfang der Verriegelungselemente 40 ein und halten diese in der Freigabestellung, bei der die Formschlusselemente 23 der Sperrsegmente 22 außer Eingriff mit den Verriegelungselementen 40 gebracht sind.

## Patentansprüche

1. Prothesenhandgelenk mit einer Basis (10) zur Befestigung an einer proximalen Prothesenkomponente und einem Schwenkteil (20), das um eine Schwenkachse (30) verschwenkbar an der Basis (10) gelagert ist,
- mit zumindest einer Befestigungseinrichtung (21) zur Festlegung einer distalen Prothesenkomponente oder Prothesenhand an dem Schwenkteil (20), wobei
- an dem Schwenkteil (20) zumindest ein Sperrsegment (22) mit daran angeordneten Formschlusselementen (23) angeordnet ist und
- an der Basis (10) zumindest ein Verriegelungselement (40) längsverschieblich entlang der Schwenkachse (30) gelagert ist, das aus einer Verriegelungsstellung, in der das Verriegelungselement (40) in Eingriff mit einem Formschlusselement (23) des Sperrsegmentes (22) steht, entgegen einer Federkraft in eine Freigabestellung bringbar ist, wobei das Verriegelungselement (40) zwei Freigabebereiche (41, 42) aufweist, mit denen eine erste und eine zweite Freigabestellung verwirklicht wird, der erste Freigabebereich (41) in Verschieberichtung beabstandet zu dem zweiten Freigabebereich (42) angeordnet ist und dem zweiten Freigabebereich (42) ein Sperrelement (50) zugeordnet ist, das das Verriegelungselement (40) in der zweiten Freigabestellung hält, **dadurch gekennzeichnet, dass** das Schwenkteil (20) entgegen einer Federkraft verschwenkbar an der Basis (10) gelagert ist und über zumindest eine in entgegengesetzte Richtungen wirkende Federkomponente (80) in einer Ausgangsstellung gehalten wird, wobei die Federkomponente (80) an einem Anschlag (288) angeordnet ist und das Widerlager (18) von dem Anschlag (288) weg außer Eingriff mit der Federkomponente (80) bringbar ist.

2. Prothesenhandgelenk nach Anspruch 1, **dadurch gekennzeichnet, dass** das Verriegelungselement (40) in der ersten Freigabestellung in die zweite Freigabestellung entgegen der Federkraft und in die Verriegelungsstellung durch die Federkraft verschiebbar ist.

3. Prothesenhandgelenk nach Anspruch 1 oder 2, **dadurch gekennzeichnet, dass** die Federkraft gegen eine Verschiebung in die zweite Freigabestellung größer als die Federkraft gegen die Verschiebung in die erste Freigabestellung ist.

4. Prothesenhandgelenk nach Anspruch 3, **dadurch gekennzeichnet, dass** die Federkraft gegen eine Verschiebung in die zweite Freigabestellung zu Beginn der zweiten Freigabebereiches (42) 10% größer als die Federkraft gegen die Verschiebung des ersten Freigabebereiches (41) am Ende der ersten Freigabestellung ist.

5. Prothesenhandgelenk nach einem der voranstehenden Ansprüche, **dadurch gekennzeichnet, dass** das Verriegelungselement (40) über zumindest ein Federelement (60) gegen eine Verschiebung in eine Freigabestellung gegenüber der Basis (10) abgestützt ist.

6. Prothesenhandgelenk nach Anspruch 5, **dadurch gekennzeichnet, dass** das Federelement (60) zweistufig, mit einer abgestuften oder progressiven Federkennlinie ausgebildet ist und/oder dass mehrere Federelemente in Verschieberichtung des Verriegelungselementes (40) nacheinander in Eingriff bringbar angeordnet sind.

7. Prothesenhandgelenk nach einem der voranstehenden Ansprüche, **dadurch gekennzeichnet, dass** dem Verriegelungselement (40) zwei einander in Verschieberichtung gegenüberliegende Betätigungselemente (70) zugeordnet sind.

8. Prothesenhandgelenk nach einem der voranstehenden Ansprüche, **dadurch gekennzeichnet, dass** zwei parallel orientierte Verriegelungselemente (40) beiderseits der Schwenkachse (30) an der Basis (10) gelagert sind.

9. Prothesenhandgelenk nach einem der voranstehenden Ansprüche, **dadurch gekennzeichnet, dass** die Freigabebereiche (41, 42) des Verriegelungselementes (40) als Abflachungen oder Durchmesserverringerungen ausgebildet sind.

10. Prothesenhandgelenk nach einem der voranstehenden Ansprüche, **dadurch gekennzeichnet, dass** das Sperrsegment (22) kreissegmentförmig ausgebildet ist.

11. Prothesenhandgelenk nach einem der voranstehenden Ansprüche, **dadurch gekennzeichnet, dass** die Formschlusselemente (23) an dem Umfang des Sperrsegmentes (22) angeordnet oder ausgebildet sind.

12. Prothesenhandgelenk nach einem der voranstehenden Ansprüche, **dadurch gekennzeichnet, dass** das Sperrelement (50) in Richtung auf das Verriegelungselement (40) federbelastet ist.

13. Prothesenhandgelenk nach einem der voranstehenden Ansprüche, **dadurch gekennzeichnet, dass** das Verriegelungselement (40) eine Formschlusskomponente (45) aufweist, mit der das Sperrelement (50) in Eingriff bringbar ist.

14. Prothesenhandgelenk nach einem der voranstehenden Ansprüche, **dadurch gekennzeichnet, dass** die Federkomponenten (80) in Ausnehmungen (28) an der Basis (10) oder dem Schwenkteil (20) angeordnet sind und sich an Widerlagern (18) an dem jeweils gegenüberliegenden Element abstützen.

15. Prothesenhandgelenk nach Anspruch 14, **dadurch gekennzeichnet, dass** das Widerlager (18) als Buchse ausgebildet ist, die an dem Sperrsegment (23) oder der Basis (10) festgelegt ist.
